# EUROPEAN PATENT APPLICATION

(11) **EP 1 457 204 A1**
(43) Date of publication of application: **15.09.2004**
(21) Application number: 02788846.0
(22) Date of filing: 16.12.2002
(51) Int. Cl.: A61K 9/26

(54) **RELEASE CONTROL TYPE FORMED PRODUCT**

(30) Priority: 19.12.2001 JP 2001385864
(71) Applicant: Sanwa Kagaku Kenkyusho Co., Ltd., Nagoya-shi, Aichi 461-8631 (JP)
(72) Inventor: OZEKI, Yuichi, SANWA KAGAKU KENKYUSHO CO., LTD, Nagoya-shi, Aichi 461-8631 (JP)
(74) Representative: Hofinger, Stephan
(86) International application number: PCT/JP2002/013154
(87) International publication number: WO 2003/051339

(57) **Abstract**

In order to provide a molded product capable of manifesting a desired release control pattern of an effective ingredient by direct tabletting method, a controlled release molded product was devised characterized in having an outer layer and a plurality of cores or a core partially provided with constrictions and portions that can be substantially identified as a plurality of cores and controlling release of the effective ingredient contained in the core portion according to the distance from the outside of the molded product to individual core. The molded product of the present invention can also be used in a dividable controlled release molded product in which the release property remains unchanged after division by providing a structure coupling a plurality of unit molded products and ensuring that the distance from the division surface to the core is sufficiently longer than the distance from the external reference plane on the molded product surface that stipulates the distance from the outside of the molded product to the core. It is possible to readily manufacture the molded product of the present invention using integral compression molding means having a double-structured punch - punch consisting of a core punch and an outer punch enclosing part or whole of the outer perimeter of the core punch - above and below a die.

## Description

### TECHNICAL FIELD

The present invention relates generally to a molded product manufactured mainly by compressing powder or granular particles as molding materials, and more particularly to a molded product in which the release of the active ingredients can be controlled.

### BACKGROUND ART

In therapeutic, recent years have seen ongoing efforts to develop solid preparations called time-specific controlled release preparations to enhance control of the absorption process such as reducing side effects and improving the targeting of absorption in the digestive system. While including sustained release and enteric preparations in broad sense, the preparations generally refer to time lag or pulsed preparations.

In the case of pharmaceutical drugs, there exist generally compounds for new drug candidates and drug substances of commercial drugs called active components (hereafter represented as "active components") having various physicochemical and biopharmaceutical properties, requiring various release control (in vitro and in vivo) technologies appropriate for the purpose of the therapy. By setting drug release patterns (dissolution patterns described later; synonym for dissolution property) adapted for the purpose of therapy, it is possible to (1) enhance the efficacy by chronopharmacotherapy and reduce side effects, (2) avoid the first pass metabolism caused by local release in the colon, rectum, etc., (3) enhance the biological applicability of peptide drugs and the like and (4) conduct localized treatment of specific areas, not only rationalizing treatment and rendering it more adequate but also leading to improved patients' QOL (Quality of Life). To establish technologies to allow control over release of such active components, therefore, needs have been voiced continuously to (1) create wide-ranging dissolution patterns, (2) build a highly accurate release control technology and (3) downsize the dissolution control technology (Kobayashi; PHARM TECH JAPAN vol. 17 No. 1 2001).

The time-controlled explosion system (TES) developed by Hata et al. (Hata, Ueda: PHARM TECH JAPAN vol. 4 1988) is among systems that release drug after a given lag time, which is one example of conventional time-specific controlled release preparations. The system has a three-layer structure requiring a drug layer, a swelling layer and a water-insoluble polymer layer. The preparation allows for rapid drug release by using a swelling agent to absorb water penetrating inside the system from outside through the polymer coating and by collapse of the polymer coating due to swelling force resulting from hydration of the swelling agent.

As another time-specific controlled release preparation, a preparation of three-layer structure, described in Japanese Patent Application Laid-Open Publication No. 7-2650, is also described as having an enteric coating as the outer layer, an inner layer thereinside for allowing sustained release and a core containing the drug. This preparation also elutes rapidly after a given time lag.

Pulsincap, developed by RP Sherer, is a preparation that achieves time-specific release control differently from the aforementioned multi-layer coating method. This preparation has a hydro-gel polymer plug fitted to an insoluble capsule, thus controlling the release time by the length of the gel. Although each of such preparations can rapidly release the active component after a given lag time, a number of problems have yet to be addressed in terms of practical use, including a special capsule required and limited volume thereof.

While an example of time-specific controlled release preparations was described above as conventional time-lag preparation, these preparations are saddled with many problems. In the case of a time-specific controlled release preparation constituted by a multi-layer coating, for instance, variations in coating thickness between layers result in variations in preparation's dissolution property. This means that unless the coating thickness is precisely controlled, it is impossible to precisely control the dissolution property. In actuality, however, as long as the manufacturing method or "coating" is used, variations in film forming are unavoidable. More specifically, film layer thickness varies from one preparation lot to another. Even one preparation has a different thickness from one area to another (in general, difference in coating thickness tends to occur between the tablet edge and the remaining areas). In light of the above, these preparations are generally often used for granules that are easier to film-coat, and one must say that applying the preparations to tablets themselves is technically difficult. Besides, stacking a number of coating layers is intricate from the viewpoint of manufacturing cost and technically difficult, making it more costly than preparations showing an ordinary dissolution.

Next, a description will be given of time-specific controlled release preparations having the release property of repeating dissolution a plurality of times (multi-pulse). The aforementioned method of repeated coating requires adjustment of coating thickness in consideration of increase in granule volume caused by coating or adjustment of main component concentration according to the coating thickness if there exists an active component thereinside. Further, the more multi-layer coatings, for example, the more difficult it becomes to downsize preparations. In other words, the more precision is required in dissolution control, the more intricate and complicated the manufacturing steps become, making the method hardly industrially viable.

Conventional controlled release preparations, in which a tablet is repeatedly coated, is further problematic in that the tablet is not dividable (i.e., tablet cannot be split). Division of tablets in the medical workplace is widely used to tailor the drug concentration to pharmacokinetics of individual patients. While a dividable tablet commonly has a concave line (scored line) on the surface and dividing the tablet along the line allows for administration of half tablet. However, a controlled release preparation produced by coating cannot maintain its controlled release properties if it is divided or split when administered. That is, dividing the tablet results in direct exposure of individual coating layers on the split surface to the outside world, making it impossible to control drug release.

On the other hand, Japanese Patent Application Laid-Open Publication Nos. 8-157392 and 2000-128779 disclose preparations that have a time lag and a plurality of pulses by adjusting, in advance, a plurality of granules having different dissolution properties, mixing the plurality of granules and filling the mixture in a capsule, etc. for use as preparation. However, not only the granules in the disclosed preparation involve variations in dissolution attributed to coating, but also it is apparent from the fact that the preparation comprises a plurality of granules having different dissolution properties that variations in dissolution become more obvious. As an alternative method, it is theoretically possible to arrange a plurality of cores in layered form by repeating the conventional method of manufacturing a press-coated tablet. That is, this method consists of manufacturing a multi-core press-coated tablet by manufacturing a press-coated tablet in advance and using the tablet as a core in a new press-coated tablet. However, the present method requires a certain size of the core tablet, making the upsizing of the tablet unavoidable. For example, if a core tablet of the first layer is 6mm in diameter with the tablet, into which the core tablet is introduced (introduced with 2mm gap on each side of the core tablet of 6mm in diameter), being 10mm in diameter and if the same method is repeated up to the third layer, the final tablet size is 14mm, making the downsizing impossible and resulting in departure from the tablet size generally said to be swallowable. Naturally, manufacturing cost becomes inevitably higher, making the method hardly feasible. Besides, the tablet is undividable due to its structure as with coated-type release preparations.

### DISCLOSURE OF THE INVENTION

In order to solve the aforementioned problems with the conventional art, the present inventor earnestly conducted studies to provide a molded product that stably offers a desired controlled release profile of the effective ingredient using a manufacturing method by simple direct tabletting rather than the coating method. As a result, they devised a molded product, noting the localization of substantially a plurality of cores containing an effective ingredient inside the molded product, in which the plurality of cores were localized at specific positions. That is, the present invention is a controlled release molded product characterized in having an outer layer and a plurality of cores, wherein the release of the effective ingredient contained in the core portions can be controlled by varying the distance from the outside of the molded product to the individual cores arranged at specific positions, or a controlled release molded product characterized in having an outer layer and a core partially provided with constrictions and portions that can be substantially identified as a plurality of cores, wherein the release of the effective ingredient contained in the core portion is controlled by varying the distance from the outside of the molded product to the individual portions that can be substantially identified as a plurality of cores arranged at specific positions. Further, in order to render dividable the controlled release molded product of the present invention, they studied the arrangement of cores and scored line so as to maintain the release property thereof after division, and as a result, arrived at devising a molded product provided, as described below, with scored line so as to ensure that the distance from the outside of the molded product to the cores remains unchanged before and after division. That is, this molded product is a dividable controlled release molded product characterized in having a structure coupling the plurality of controlled release molded products of the present invention and being dividable along the coupling portion as division surface, with the distance from the division surface to any core in individual unit molded products being sufficiently longer than the distance from the external reference plane on the molded product surface that stipulates the distance from the outside of the molded product to cores.

The controlled release molded product of the present invention can be readily manufactured using integral compression molding means having double-structured punches consisting of a core punch and an outer punch enclosing part or whole of the outer perimeter of the core punch.

For example, a molded product can be transformed into a controlled release molded product having a plurality of given release peaks by linearly scattering cores from the outside of the molded product toward the inside thereof and allowing the outer layer and/or cores to dissolve successively from the outside because the molded product erodes from the outside toward the inside.

### BRIEF DESCRIPTION OF THE DRAWINGS

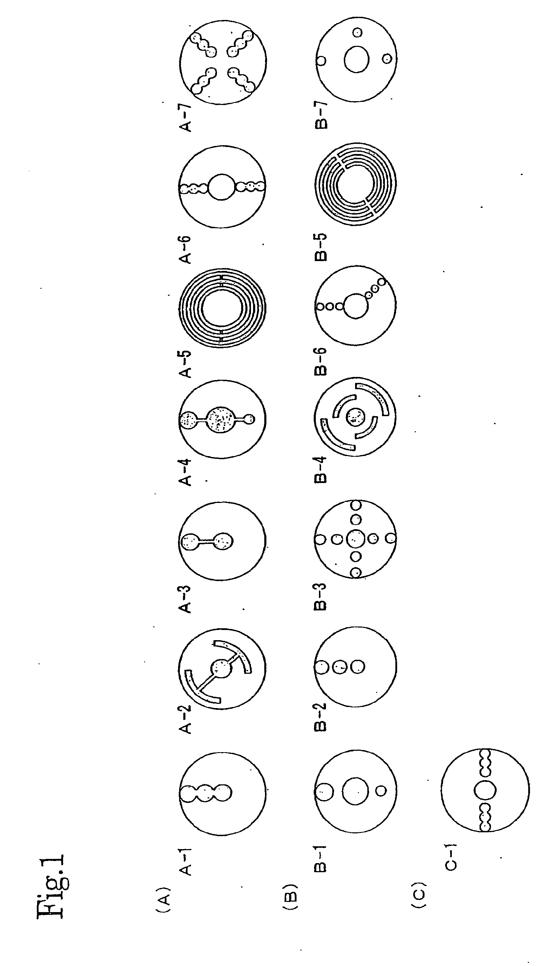

Figs. 1A to 1C illustrate variations of a controlled release molded product according to the present invention in which sectional views of the finished molded products are shown. The shaded areas represent the cores and the remaining areas representing the outer layer, Figs. 1A-1 to 1A-7 are examples of molded products having a core partially provided with constrictions and portions that can be substantially identified as a plurality of cores. Figs. 1B-1 to 1B-7 are examples of molded products having a plurality of cores, and Fig. 1C-1 is an example of molded product in which cores of the types shown in Figs. 1A and Figs. 1B are used in conjunction; these figures also constitute, as such, examples of tip portions of double-structured split punch used to manufacture the molded product of the present invention, and in that case, the shaded areas represent a core punch, and the remaining areas representing an outer punch;

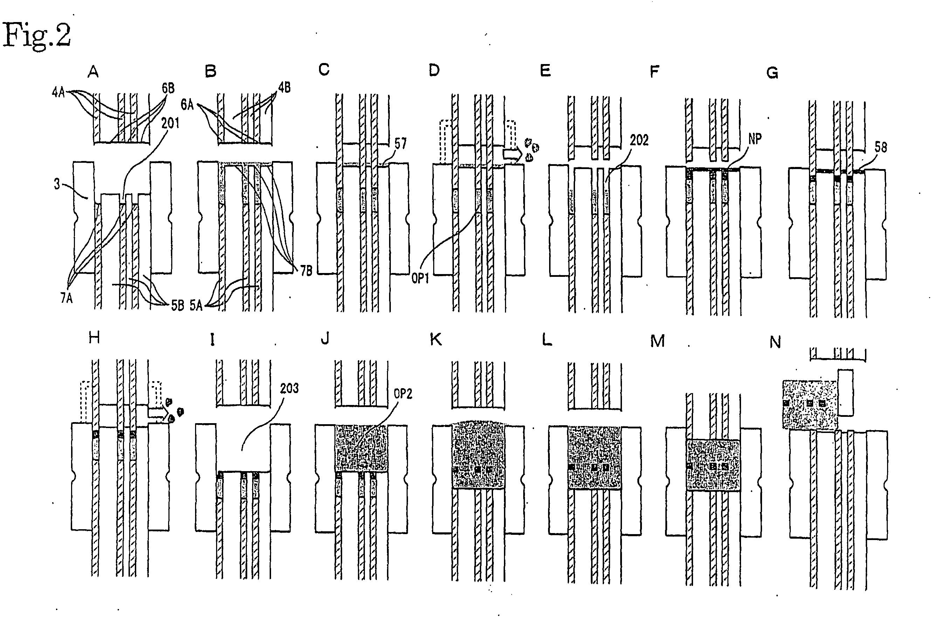

Figs. 2A-2N illustrate explanatory views of punch tip operations showing an example of a manufacturing method of the controlled release molded product of the present invention, with only the core punch shown by oblique lines and oblique lines as cross section omitted for convenience;

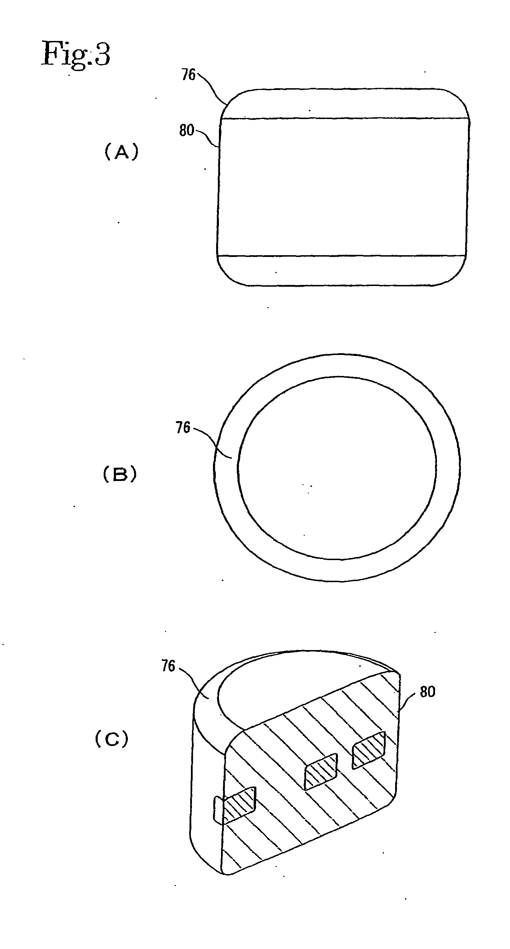

Figs. 3A to 3C illustrate examples of the controlled release molded product of the present invention, Fig. 3A being a side schematic view, Fig. 3B being a top view, and Fig. 3C being a perspective view of the cross-section of the molded product;

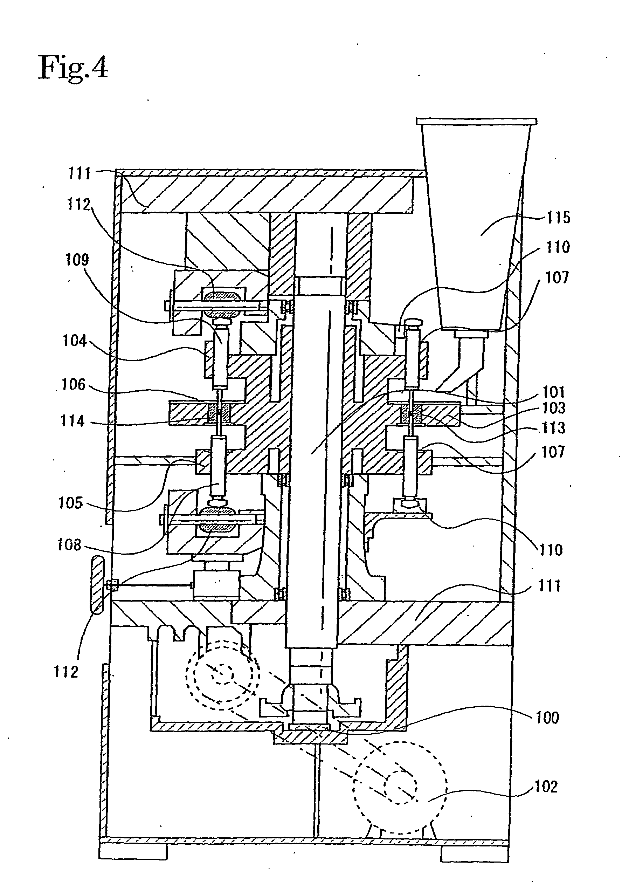

Fig. 4 is an overall front sectional view of a common rotary compression-molding machine, except that the sectional views of the punch, the vertical shaft, and the hopper are not shown;

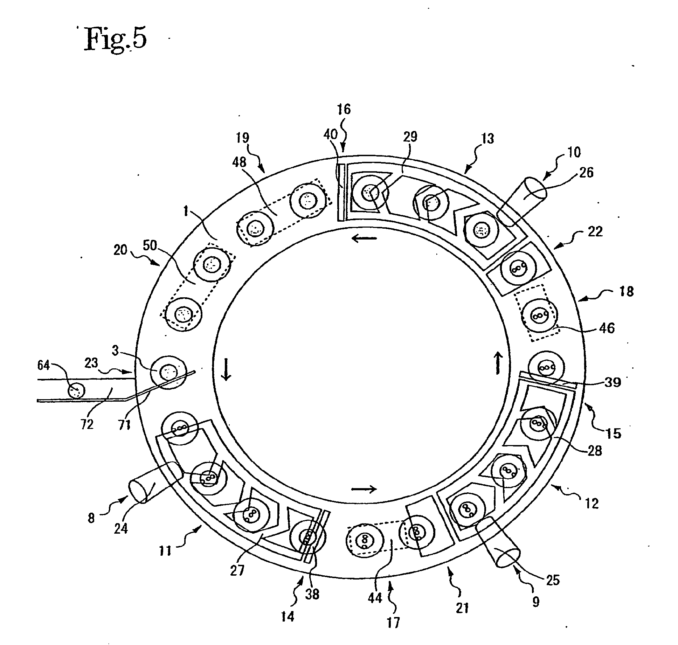

Fig. 5 is a schematic plan view showing the top side of a turntable in an embodiment of the rotary compression-molding machine capable of manufacturing the controlled release molded product of the present invention;

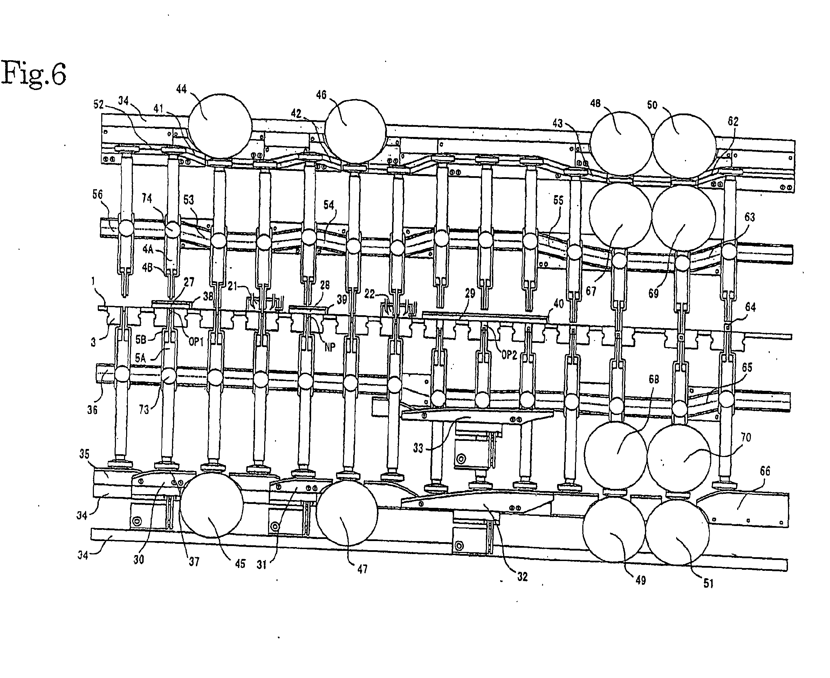

Fig. 6 is a schematic view including a partial sectional view and showing the operational mechanism of upper and lower punches by developing the turntable in an embodiment of the rotary compression-molding machine capable of manufacturing the controlled release molded product of the present invention;

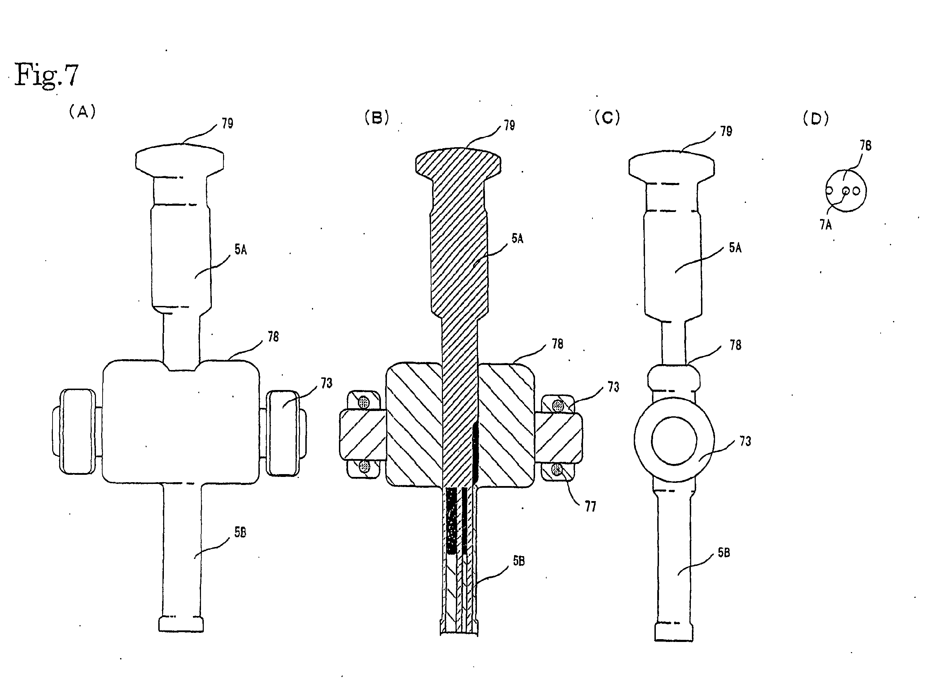

Figs. 7A to 7D illustrate an example of a double-structured punch (lower punch) capable of manufacturing the controlled release molded product of the present invention, Fig. 7A being a front schematic view, Fig. 7B being a front sectional view, Fig. 7C being a side view, and Fig. 7D being a front view of the punch tip;

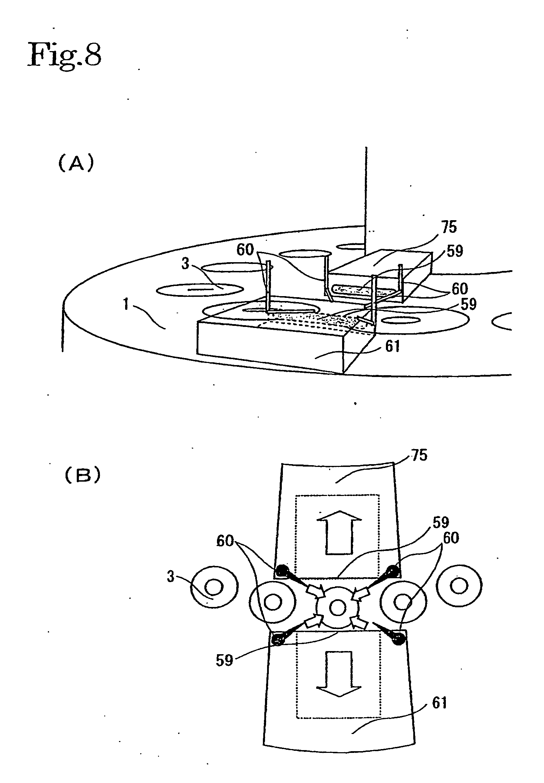

Figs. 8A and 8B illustrate a residual powder or granular particles removal device of the present invention, Fig. 8A being a bird's-eye view, and Fig. 8B being a top view;

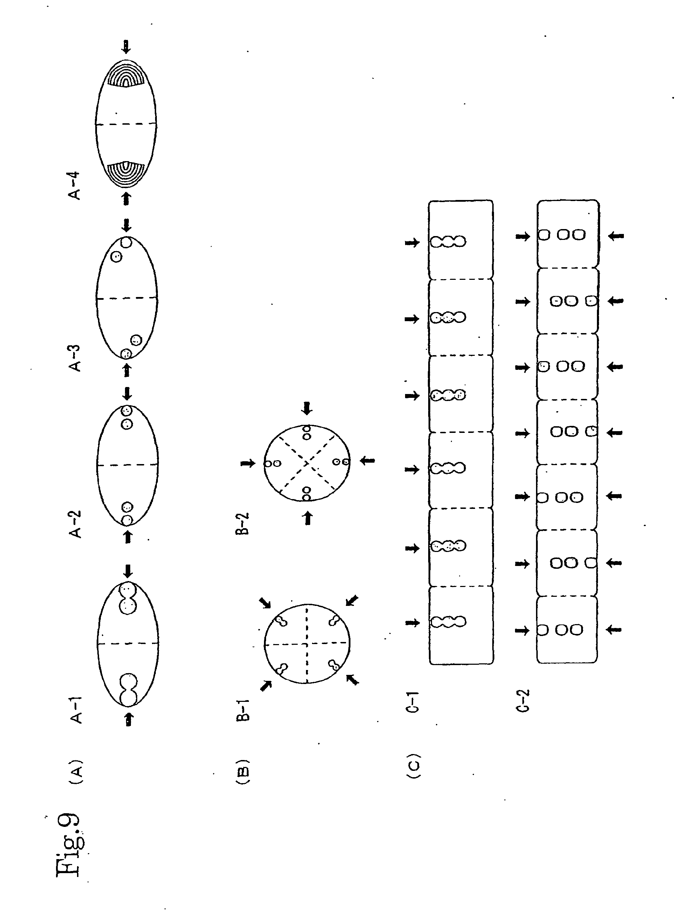

Figs. 9A to 9C illustrate variations of the controlled release molded product of the present invention in which sectional views of the finished molded products are shown, with the shaded areas representing the cores and the remaining areas representing the outer layer, and the dashed lined representing the division surface. The arrows represent the external reference plane. Figs. 9A-1 to 9A-4 show examples of molded products that will be divided into two parts, Figs. 9B-1 and 9B-2 being examples of molded products that will be divided into four parts, and Fig. 9C-1 and 9C-2 being an example of a continuous body of dividable controlled release molded products; these figures also constitute, as such, examples of embodiments of tip portions of double-structured split punch used to manufacture the molded product of the present invention, and in that case, the shaded areas represent the core punch, the remaining areas represent the outer punch, and the dashed lines represent convex lines for forming scored lines.

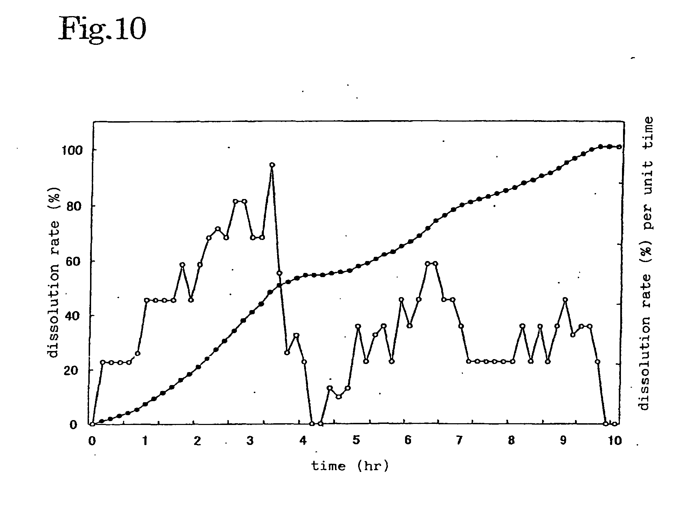

Fig. 10 is a dissolution pattern diagram for a controlled release molded product, in pure water, of manufacturing example 1 using hydroxypropylcellulose for the outer layer, the closed circles representing the dissolution rate (%) of thoeophyline, and the open circles representing the dissolution rate (%) of thoeophyline per unit time; and

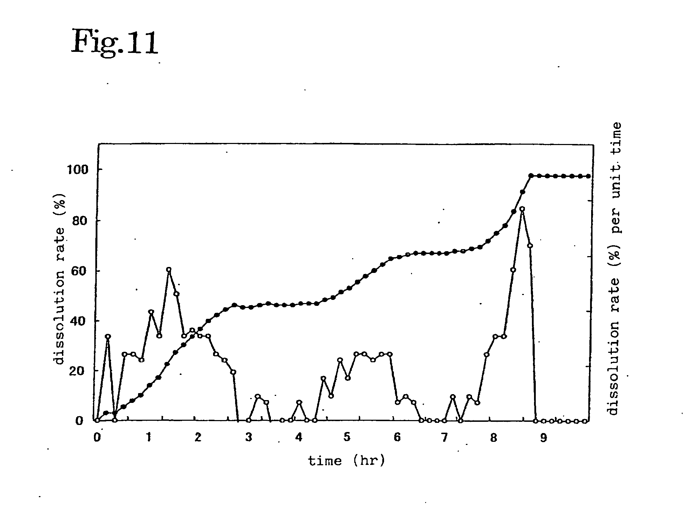

Fig. 11 is a dissolution pattern diagram for a controlled release molded product, in test solution No. 2 (ph6.8), of manufacturing example 2 using Eudragit for the outer layer, the closed circles representing the dissolution rate (%) of thoeophyline, and the open circles representing the dissolution rate (%) of thoeophyline per unit time.

### BEST MODE FOR CARRYING OUT THE INVENTION

In this description, not only the active ingredient in drugs (effective ingredient, main ingredient) and main ingredient in foods but also any ingredient for which release from a molded product is controlled is encompassed by the term "effective ingredient" whereas ingredients other than effective ingredient, namely, various additives such as filler, binder, disintegrator, lubricant and anti-agglutinator that are regularly used in the fields of formulation technology and molded product manufacturing technology are collectively referred to as "filler ingredients."

The controlled release molded product of the present invention is characterized in having an outer layer and substantially a plurality of cores whereby the release of the effective ingredient contained in the core portions is controlled according to the distance from the outside of the molded product to individual portions arranged at specific positions. The term "substantially a plurality of cores" may be either a plurality of cores or a core partially provided with constrictions and portions that can be substantially identified as a plurality of cores.

Here, the term "a plurality of cores" refers to a plurality of cores that are literally separated from one another (refer to Figs. 1B1 to 1B7). On the other hand, the term "core partially provided with constrictions and portions that can be substantially identified as a plurality of cores" refers, in the present description, to embodiments as shown in Figs. 1A1 to 1A7, which is, namely, the case of a core that, while being a single connected core if viewed as a single molded product, appears to be several cores coupled together as a result of "constrictions" present in the core shape. In the case of such a core, there are a plurality of peaks in the effective ingredient release profile in the actual administration environment due to substantial isolation between cores or change in release rate, for example, as a result of swelling of the outer layer ingredient around the cores.

It should be noted that there may be, in a molded product, a single core or a plurality of cores that are partially provided with constrictions and portions that can be substantially identified as a plurality of cores (refer to Figs. 1A1 to 1A7). Further, such cores may coexist with other ordinary cores (refer to Figs. 1C-1). The present invention includes all these embodiments. It should be understood that the same holds true for the dividable controlled release molded product described later.

In the present invention, the distance from the outside of the molded product to a core or portion that can be substantially identified as a core (hereinafter collectively referred to as "core") is the shortest distance from the outside of the molded product to such a core. The molded product surface, or the starting point thereof, that determines this shortest distance is assumed to be the external reference plane that stipulates the distance from the outside of the molded product to cores. This external reference plane may be viewed not only as a plane but also as a point and may also exist in a plurality. In the present invention, cores are arranged inside the molded product including the molded product surface. The arrangement of the cores is determined by the intended release profile. That is, the release profile of the molded product of the present invention is determined by positions at which individual cores are arranged. This means that the first release occurs from the core closest to the outside of the molded product, followed successively by those toward the inside of the molded product. In the case of a donut-shaped molded product in troche form, for example, external reference planes often exist not only on the outer perimeter surface outside the molded product but also at hole portions thereinside. Since core-to-core release time lag depends on the difference in distance from the outside of the molded product to each individual core, the larger the difference in distance, the longer the time from the first to next release. Here, core positions can be freely set according to the desired release profile. Needless to say, the number of release peaks depends on the number of cores. While specific examples of core arrangement are shown in Figs. 1, there are an indefinite number of arrangement patterns, including those in which a plurality of cores are linearly scattered from the outside of the molded product toward the inside thereof with one external reference plane (e.g., typically Figs. 1A-1 and 1B-2) and others in which cores are arranged from several directions with a plurality of external reference planes (e.g., typically Figs. 1B-4 and 1B-7).

The dividable controlled release molded product of the present invention is characterized in having a structure in which a plurality of controlled release molded products are coupled together and dividable along the coupling portion as the division surface, with the distance from the division surface to any core in the individual unit molded products being sufficiently longer than the distance from the external reference plane on the molded product surface that stipulates the distance from the outside of the molded product to cores. The reason lies in that, in the presence of a core whose distance to the division surface is not sufficiently longer than the distance from the external reference plane, the external reference plane of the core becomes a division surface as a result of division, possibly changing the shortest distance from the outside of the molded product. Since a molded product cannot always be divided uniformly, a "sufficiently long" configuration is needed to allow for those variations. Here, the term "sufficiently long", while being dependent on the molded product size, is probably, for example, at least over 1mm longer, and preferably at least over 2mm longer. Thus, the release profile of individual molded products after division (unit molded products) maintains the same profile as with the release profile of the controlled release molded product before division. It should be noted that the term "having a structure coupling the plurality" does not mean that the molded product was manufactured by actually coupling individual unit molded products together. Rather, it denotes that individual unit molded products are appear to be coupled together. Similarly, the term "coupling portion" is not the coupling surface created by actual coupling but the apparent coupling surface anticipated as the boundary surface of a unit molded product. The dividable controlled release molded product of the present invention preferably has a concave scored line at the division position and can be divided at the scored line into separate unit molded products.

The Embodiments as shown in Figs. 9 are examples of dividable controlled release molded products. Figs. 9A-1 to 9A-4 are examples of molded products from which two controlled release molded products can be obtained as a result of division from the division surface (division line portion) shown by a dashed line, whereas Figs. 9B-1 and 9B-2 are examples of molded products that are similarly dividable into four parts. Figs. 9C-1 and 9C-2 are continuous bodies of controlled release molded products arranged linearly and continuously, constituting examples of molded products dividable into individual unit molded products at coupling portions having a division line. It should be noted that the external reference planes are indicated by arrows in Figs. 9. In all post-division and pre-division molded products, the shortest distance from the outside of the molded product to the core is the distance from these external reference planes. Although only natural, there may be, in the present dividable controlled release molded product, a plurality of cores, a core partially provided with constrictions and portions that can be substantially identified as a plurality of cores or both thereof coexisting with each other.

In the present invention, it is further possible to control release of the effective ingredient by selecting an appropriate filler ingredient. That is, in addition to release control by the distance from the outside of the molded product to the core containing effective ingredient, release control by filler ingredient can be concurrently used. Release control by selection of a filler ingredient not only controls release rate of the effective ingredient and release time lag by the filler ingredient property but also plays an important role, as described later, in a core partially provided with constrictions and portions that can be substantially identified as a plurality of cores. Here, the filler ingredient controlling release rate of the effective ingredient can be contained in the core or the outer layer.

For example, use of an effective ingredient and sustained release filler ingredient in the core adjusts the diffusion speed of the effective ingredient and also makes it possible to switch each pulse release to sustained release. Further, by using a filler ingredient low in solubility for the outer layer, it is possible to prolong the release time lag between pulses.

When a matrix-type sustained release base is used primarily as filler ingredient in the outer layer, embodiments as those of the present invention are particularly useful that use a core partially provided with constrictions and portions that can be substantially identified as a plurality of cores. In general, as far as the release pattern is concerned, a molded product consisting of a matrix type sustained release base and effective ingredient diminishes in size as a result of gradual erosion as the molded product surface becomes gelated without suddenly collapsing as with ordinary tablets. Therefore, when the base is used, the constricted portions of the core are brought closer to each other or bonded together as a result of swelling or gelation of the outer layer ingredient surrounding the core due to water absorption or a combination thereof, thus allowing for the core to serve the function similar to that of a plurality of cores. That is, after absorbing water, the matrix type sustained release base prevents sudden release of the effective ingredient and switch the release to slow one (sustained release) due to this property. Among matrix type sustained release bases are hydroxypropylcellulose, hydroxypropylmethylcellulose, methylcellulose, carboxy methylcellulose sodium - cellulose derivertives - and polyvinyl alcohol. These ingredients can be used alone or in combination with each other and may be used concurrently with an ordinary filler ingredient.

Further, the use of a base with the above property in an ordinary molded product having a plurality of cores provides a preparation having a plurality of release pulses as the overall release pattern while at the same time ensuring sustained release of drug within the matrix. Conversely, to release the core ingredient by dissolving the molded product gradually from the outside, it suffices to use a slow-dissolving polymer for the outer layer, including such ingredients as natural polymers, synthetic polymers and synthetic elastomers (p.36 Practical Drug Delivery System by Kohei Miyao). For example, poly lactic acid and copolymer of lactic acid-glycolic acid are among them. These ingredients can, as with the matrix bases, be used alone or in combination with each other and may be used concurrently with an ordinary filler ingredient.

The outer layer ingredient used in the molded product of the present invention thus has a considerable impact on the release pattern of the effective ingredient from the cores. On the other hand, the cores of the present invention are generally assumed to contain an effective and filler ingredients or substantially an effective ingredient alone. As described earlier, while a similar sustained release filler ingredient, as illustrated in relation to the outer layer, may be used in the cores, it is preferred that effective ingredient release from the cores be fast, since the molded product of the present invention has its release controlled by the positioning of individual cores. Among ingredients that allow fast release are sugars typified by lactose and crystalline cellulose that serves also as a disintegrator. Conversely, it is not preferred for the cores to contain a filler ingredient having the property of delaying effective ingredient release from the cores, since these results in overlap with release as controlled by the positioning of the core and therefore makes it difficult to obtain the intended release profile. It should be noted that the term "release delay", although not precisely definable because the release rate varies due, for example, to the release property of the effective ingredient itself, is defined in the present description as intentional delay of the release rate of the effective ingredient itself, whereas the term "filler ingredient having the property of delaying release" denotes an ingredient capable of delaying the release rate of the effective ingredient by the filler. In actuality, while filler ingredients include polymers such as the aforementioned matrix type sustained release bases, they are not limited thereto.

A description will be given below of application of the present invention primarily to pharmaceutical drugs.

It suffices for the cores (including the portions that can be substantially identified as cores) in the present invention to be shaped and sized dependening primarily on the intended release profile and release amount. While it is preferred to reduce the size in terms of preventing upsizing of the molded product as a whole, exceedingly small cores are not preferred either, as downsizing the cores more than necessary can readily create a bottleneck to smooth charging of the core ingredients in the molding step and physical property limitations of powder or granular particles for the core ingredients become more rigorous. After all, in the case of round cores, it suffices to design them to be 3mm to 0.5mm in diameter and preferably 2mm to 1mm in diameter. Next, the shape of cores is not limited, and it suffices to shape them so as to fit the intended release pattern. While various combinations may be possible such as identical shape and size for all cores in the molded product and different shapes and sizes therefor, it is preferred that they be shaped in consideration of difficulties involved in machining the punch tip shape of the punch described later in relation to the manufacturing method or ease of powder or granular particles charging in the manufacturing steps.

On the other hand, while the shape and size of the controlled release molded product of the present invention are affected by the shape and size of the cores, it is preferred that the molded product be shaped and sized so as to be easy to hold or swallow. The shape thereof is not specifically limited, but it is preferred that the molded product be a round or oval preparation notably in pharmaceutical drugs. As for the size thereof, on the other hand, it suffices, in the case of a round tablet, to design the tablet to be, for example, 13mm or less in diameter or 4mm to 13mm in diameter, preferably 5mm to 11mm in diameter and more preferably 6mm to 9mm in diameter.

Various additives may be used for the cores and the outer layer of the controlled release molded product of the present invention including filler, binder, disintegrator, lubricant and anti-agglutinator (already defined collectively as "filler ingredients") that are regularly used in the fields of formulation technology, although this partly overlaps with the previous description. As for the addition amount thereof, such additives can be used without any problem in amounts based on the knowledge regularly used in the field of formulation technology. Effective ingredient can be added to not only the cores but also the outer layer, thus making it possible to provide a release pattern having release peaks in a scattered manner while continually maintaining effective ingredient release or a special molded product that contains different effective ingredients in the outer layer and the cores.

Wide-ranging pharmaceutical drugs can be contained in the outer layer and the cores of the controlled release molded product of the present invention as an effective ingredient. Among pharmaceutical drugs for exploiting the release property of the preparation are nifedipine, a preventive drug for angina pectoris attack (chest pain) in the early morning, and morphine sulfates, cancer pain treatment drugs of which continuous drug release is demanded. However, it is not always necessary to select a pharmaceutical drug in consideration of the release property of the preparation, and any pharmaceutical composition may be chosen as long as oral administration is possible.

While ingredients to be contained in the outer layer and the cores may be used as is, granular substance may be prepared once by granulation by a normal method and sized as necessary for use. It is also possible to prepare granular substance by coating a drug's active ingredient, functional food ingredient or ordinary food ingredient together with a binder on an inactive carrier. Further, granular substance may be coated as necessary with sustained release coating, time-lag coating, enteric coating, gastric coating, water soluble coating, sugar coating, etc.

It should be noted that the controlled release molded product of the present invention is not limited to drugs and can be applied to the fields of foods, sanitary products and so on. It suffices to determine such as the size/shape of the cores or the entire molded products, ingredients to be contained and so on according to individual fields and purposes. The controlled release molded product of the present invention is not limited to those that can be orally ingested and would find application, for example, in the field of sanitary products, to a molded product controlling effective ingredient release in a solution environment.

A detailed description will be given next of a method of manufacturing the controlled release molded product of the present invention.

It should be noted that, in the present description, the term "powder or granular particles" is used to refer to all molding materials including powders and granules, except where the term "powder" is particularly commonly used.

The controlled release molded product of the present invention can be manufactured by compression molding means provided with a die having punches thereabove and therebelow, each of the punches having a double structure (details shown in the punch structure described later) consisting of a core punch and an outer punch enclosing part or whole of the outer perimeter of the core punch and both the core and outer punches being slidable and manipulatable for compression operation. That is, it is possible in the present invention to compression-mold a press-coated type molded product using only a set of die and punches through a series of steps. For this reason, the molding method employed in the present invention is called integral molding method. The term has a meaning in contrast with conventional press-coated molded products manufactured by molding a core in advance and supplying it in the middle of molding step.

The present integral molding method keeps the core in position and allows for their accurate localization, thus eliminating problems such as variations in the release pattern as a result of core "displacement." That is, the controlled release molded product of the present invention can also be worded as a "set of controlled release molded products, free of core "displacement", whose usefulness becomes apparent when grasped as a set of many." In the present description, the term "set" conveys a meaning of a number of mass-produced molded products and is more specifically 100 pieces or more or 1000 pieces or more under certain circumstances or can be 10000 pieces or more. The method of the present invention can freely change the size/shape of the core, making it possible to control release dependently on the core size/shape, namely, control the release amount of the effective ingredient dependently on the core. While maintaining the constant concentration of the effective ingredient in a plurality of cores, it is also possible to change the release amounts or release profile of the effective ingredient from individual cores by the core size and shape alone.

Next, a detailed description will be given of an example of an embodiment deemed most preferred as the method of manufacturing the controlled release molded product of the present invention mainly with reference to Figs. 2. Wordings such as a powder or granular particles for a first outer layer OP1 and a powder or granular particles for a second outer layer OP2 are not used to mean different powder or granular particles but used for convenience to make a distinction between the portions. It should be noted that the term "core" includes the plurality of cores and/or the core partially provided with constrictions and portions that can be substantially identified as a plurality of cores.

First, with lower core and outer punches 5A and 5B lowered (Fig. 2A), the lower outer punch 5B is raised while supplying a powder or granular particles for the first outer layer OP1, filling a first outer layer space 201 partially enclosed by the lower outer punch 5B and above the lower core punch 5A, with the powder or granular particles for the first outer layer OP1 (Fig. 2B). After raising the lower core punch 5A as necessary and thus discharging the excess powder or granular particles for the first outer layer OP1 out of a die 3, an upper core punch 4A and the lower core punch 5A are moved to engage each other for temporary compression (Fig. 2C), thus temporarily molding the first outer layer. (Outer layer molding step)

Next, with the temporary molded product of the first outer layer OP1 held by the lower core and outer punches 5A and 5B, a powder or granular particles for cores NP is supplied into a core space 202 partially enclosed by the lower outer punch 5B and above the temporary molded product of the first outer layer OP1 by lowering the lower core punch 5A as necessary (Figs. 2E and 2F). Then, after raising the lower core punch 5A as necessary and thus discharging the excess powder or granular particles for the cores out of the die 3, the upper and lower core punches 4A and 5A are moved to engage each other for temporary compression (Fig. 2G), thus temporarily molding the temporary molded products of the first outer layer and the core. (Outer layer/core molding step)

Further, with the temporary molded products of the first outer layer and the core held by the lower core and outer punches 5A and 5B, a powder or granular particles for the second outer layer OP2 is supplied into a second outer layer space 203 above and around the temporary molded products of the first outer layer and the core within the die 3 (Figs. 2J and 2K) by lowering the lower punch (both the lower core and outer punches 5A and 5B or the lower outer punch 5B) (Fig. 2I). The temporary molded products of the core held on the temporary molded products of the first outer layer are allowed to be partially covered with the powder or granular particles for the outer layer and the temporary molded products of the outer layer (Fig. 2K), and the excess powder or granular particles for the second outer layer OP2 is discharged as necessary out of the die 3 (Fig. 2L). It should be noted that the powder or granular particles for the second outer layer OP2 can be supplied after sufficiently lowering the lower outer punch 5B first such that the temporary molded products of the first outer layer and the core are apparently pushed up. Then, the upper punch (both the upper core and outer punches 4A and 4B) and the lower punch (both the lower core and outer punches 5A and 5B) are moved to engage each other for precompression (temporary compression) of the entire molded product consisting of the first outer layer, the cores and the second outer layer as necessary, eventually followed by main compression (Fig. 2M). (Overall molding step)

The step shown in Fig. 2N is for ejecting the completed molded product.

It should be noted that there is an alternative method of supplying the powder or granular particles for the first outer layer into a space created by lowering the lower core punch 5A alone without moving the lower outer punch 5B.

It should be noted that outer punch tip portions (6B, 7B) correspond to a circumferential edge 76 of a completed molded product shown in Figs. 3 and may be flat depending on the embodiment of the molded product. If they are not flat as shown in Figs. 2, it is preferred, to prevent contamination between the powder or granular particles for the outer layer and the cores, that steps (Figs. 2D and 2H) of removing residual powder or granular particles 57 and 58 remaining on an upper surface 7B of the lower outer punch be further added after supply of the first outer layer OP1, during compression molding thereof (during temporary molding) or thereafter and after supply of the cores NP, during compression molding of the first outer layer OP1 and the cores NP (during temporary molding) or thereafter. The present removal steps can be carried out with compressed air injection and suction, brushing, scraping, etc. or a combination thereof. These are referred to as residual powder or granular particles removal means.

In addition to the above, a variety of embodiments would be possible for the method of manufacturing the controlled release molded product of the present invention. For example, there is a method of temporarily molding, with the powder or granular particles for the fist outer layer OP1, a pot-shaped molded product first that can accommodate the powder or granular particles for the plurality of cores NP, introducing a given amount of the powder or granular particles for the cores NP into the pot-shaped molded product and further covering the powder or granular particles for the cores NP with the powder or granular particles for the second outer layer OP2, followed by compression. Alternatively, it would be possible, for example, to temporarily mold the powder or granular particles for the cores alone and hold the temporary molded product by the upper core and outer punches first, then place the core on top of the lower punch (the lower core and outer punches) filled with the powder or granular particles for the first outer layer OP1 and finally cover the core on all sides and on the top with the powder or granular particles for the second outer layer OP2, followed by main compression.

For details regarding the above alternative embodiments, please refer to the manufacturing methods, described in International Publication No. WO/O1/98067, for which the present inventors applied for a patent earlier. It should be noted that while these manufacturing methods differ from the aforementioned example in punch operation method, powder or granular particles supply/charging timings and so on, they can be carried out with punches and die similar to those used in the first example of the manufacturing method.

The method of manufacturing the controlled release molded product of the present invention can be basically readily carried out using a hydraulic press, etc. if the upper and lower punches and die as described earlier are available. The manufacturing method can be readily implemented by performing, in accordance with the sequence of steps of the present invention, a series of steps - steps of manually and/or automatically moving the upper and lower punches or the core and outer punches to predetermined positions, charging intended powder or granular particles and pressing the powder or granular particles so as to sandwich them from above and below with a hydraulic press in accordance with the sequence of steps of the present invention. In addition thereto, the manufacturing method can be carried out using the apparatus for manufacturing a press-coated molded product as described below.

The apparatus for manufacturing the controlled release molded product of the present invention has adopted the basic parts of the mechanism and construction of previously common rotary compression molding machine. Namely, the machine has a rotatable turntable, provided with a die having die holes on the turntable and being designed to compress powder or granular particles charged into the die by holding upper and lower punches above and below the die so as to be vertically slidable, moving the upper and lower punches to engage each other and pressing the powder or granular particles with the punch tips left inserted in the die. Further, the apparatus uses, for the upper and lower punches, a double punch having a double structure consisting of a core punch and an outer punch enclosing part or whole of the outer perimeter of the core punch, with both the core and outer punches being slidable and manipulatable for compression operation, comprises means for moving the core and outer punches of the double punch and means for allowing manipulation of the core and outer punches for compression operation and is constructed so as to perform the aforementioned manufacturing method through a series of steps.

That is, when the invention is embodied in a rotary compression molding machine characterized in having a rotatable turntable provided with a die having die holes on the turntable, the powder or granular particles charged into the die are compressed by holding upper and lower punches above and below the die so as to be vertically slidable, moving the upper and lower punches to engage each other and pressing the powder or granular particles with the punch tips left inserted in the die. The machine would include, for both the upper and lower punches, a double punch having a double structure consisting of a core punch and an outer punch enclosing part or whole of the outer perimeter of the core punch, with both the core and outer punches being slidable and manipulatable for compression operation. A means for moving the core and outer punches of the double punch and means for allowing manipulation of the core and outer punches for compression operation is also provided. The machine also provides, on the same turntable, supply/charging portions respectively for powder or granular particles for the outer layer and the cores, compression molding portions for the powder or granular particles for the cores and/or the outer layer and compression molding portion for the entire molded product. In the present manufacturing apparatus, there are generally two or more supply/charging portions for powder or granular particles for the outer layer.

It should be noted that a residual powder or granular particles removal device may be provided for removing powder or granular particles remaining on the lower outer punch and/or the molded product, depending on the shape of the punch tips.

The present manufacturing apparatus, if described more specifically, is a rotary compression molding machine characterized in comprising a portion for supplying a first powder or granular particles into a space roughly enclosed by the lower outer punch and above the lower core punch, a portion for subsequent compression molding of the first powder or granular particles by the upper and lower core punches, a portion for supplying a second powder or granular particles into a space roughly enclosed by the lower outer punch and above the lower core punch (or above the molded product), a portion for subsequent compression molding of the second powder or granular particles by the upper and lower core punches, a portion for supplying a last powder or granular particles into a space within the die and a portion for subsequent compression molding of the entire molded product by the upper and lower core and outer punches.

To describe, in further detail, the apparatus for manufacturing the controlled release molded product of the present invention, descriptions will be given in succession beginning with conventional rotary compression molding machine.

If the machine is shaft-driven, for example, a rotary compression molding machine has a vertical shaft 101, supported by a bearing 100, arranged at the center portion of a main body frame 111, with a motor 102 transmitting rotational drive force to the vertical shaft and a turntable 103 - splittable into two functional parts - fixed near the vertical shaft, as shown in Fig. 4. Further, there are provided an upper punch holding portion 104, located on the upper portion of the turntable, for holding an upper punch so as to be vertically slidable and a lower punch holding portion 105, located on the lower portion of the turntable, for holding a lower punch so as to be vertically slidable such that the turntable 103 is sandwiched between the upper and lower punch holding portions 104 and 105. On the turntable 103, there is a die portion made up of a plurality of die mounting holes 106, for fitting the die 114 so as to be detachable/reattachable, that are provided along the same circumference. On each of the upper and lower punch holding portions 104 and 105, there are a plurality of punch holding holes 107 drilled respectively for holding the upper and lower punches so as to be slidable. Each of the die mounting holes 107 and the punch holding holes 106 is drilled on the turntable such that the lower punch 108, the upper punch 109 and the die 114 are arranged vertically with their center lines aligned. Rails 110 are provided correspondingly for rail contact portions of the upper and lower punches 109 and 108, and the punches move vertically on the rails as they engage with and are guided by respective cams, which will be discussed later. The die 114 has a die hole 113 cut vertically through the die into which the tips of the upper and lower punches 109 and 108 are inserted. It should be noted that 112 represents a compression roller while 115 a hopper in Fig. 4.

In addition to shaft-driven rotary compression molding machine, there are other types thereof such as external gear-driven (external gear type) and internal gear-driven (internal gear type) rotary compression molding machines in which rotational drive force is transmitted by equipping the turntable with a gear.

Next, a description will be given of the double-structured punch used in the present invention and the portions associated therewith.

The double punch used in the present invention has a core punch and an outer punch enclosing part or whole of the outer perimeter of the core punch, with the outer shape of the outer punch being approximately identical to the inner shape of a die except for those portions where the core punch is not enclosed by the outer punch. Further, both the core and outer punches are slidable and manipulatable for compression operation. Here, the core and outer punches are basically slidable independently of each other, except for those portions that slide by coordination of the two punches. It should be noted that a single core punch is branched at the tip rather than having independent punches. Such a punch is preferred because it is possible to make the amount of pressure transferred and the pressure transfer speed the same during compression in order to maintain the same moldability for a plurality of cores. Further, the punch is also advantageous in terms of punch manufacture and operation because it does not result in a complicated form due to its structure. It is also possible to use, for the punch, an integrally structured shell portion separate from the tip portions all of which can be integrated into one piece by fastening them. Although not preferred, it is possible to use a plurality of independent core punches rather than branch the tip of a single core punch.

As specific examples of punch tips, the shapes shown in Figs. 1 and 9 reflect the tip shapes as is, however, when viewed in this manner the shaded areas represent the core punch and the remaining areas correspond to the outer punch. To provide a scored line at the division surface on the molded products in Figs. 9, a scored line in the form of a concave recess can be formed on the molded product surface by using a punch provided with a convex line on the punch surface. It should be noted that a scored line can be provided on either the upper or lower punch or both thereof.

An example of punch can be exemplified by a punch having a structure as shown in Figs. 7, which corresponds to the punch shown in Fig. 6. This punch has the core and outer punches 5A and 5B, an outer punch compression head 78, a core punch compression head 79 and an outer punch vertical sliding motion adjustment roller 73. In the compression molding step, compression of the core portions is carried out mainly by pressing the core punch compression head 79 with compression rollers (45, 47, 49, 51 in Fig. 6) whereas compression of the portions other than the core portions is performed by pressing the outer punch compression head 78 with compression rollers (68, 70 in Fig. 6). This allows for compression operation using the core and outer punches.

while the vertical sliding motion of the core punch is controlled by a normal method mainly using the core punch rail and a core bottom portion 37 (same portion as the core punch compression head 79), the vertical sliding motion adjustment roller 73 is provided that comes in direct contact with the outer punch rail to allow vertical sliding motion of the outer punch. Preferably, a plurality of bearings 77 are provided within the rollers to allow rotation of the rollers and smooth vertical sliding motion of the outer punch.

Here, the vertical sliding motion adjustment roller 73 is arranged outside the outer punch compression head 78, with the vertical sliding motion adjustment roller 73 separated from the outer punch compression head 78. This allows the compression roller to apply pressure only to the outer punch compression head 78 while not applying direct pressure to the vertical sliding motion adjustment roller 73, thus preventing damage to the bearings 77 within the vertical sliding motion adjustment roller 73. In compression operation, it is possible to apply pressure to the outer punch more from the side of the core punch, thus allowing efficient transfer of pressure from the compression roller to the powder or granular particles. Contact portions of the compression rollers of the core and outer punches (the outer punch compression head 78 and the core punch compression head 79) are vertically separated from each other, thus preventing interference between the compression rollers of the core and outer punches.

While Figs. 7 assume the lower punch, the same holds true for the upper punch. Among differences between the upper and lower punches are a shorter length of the tip portion of the upper punch inserted into the die and different portions prescribing the punch motion (e.g., spaces within the punches) due to difference in motion between the upper and lower punches.

Alternatively, the double punch used in the present invention may be that in which the motions of the core and outer punches are respectively controlled in reverse. That is, the punch controls the motion of the core punch with the vertical sliding motion adjustment roller and the rail and the motion of the outer punch with the punch bottom portion and the rail. A description of the punch will be omitted since the same holds true for the punch as for the punch of Figs. 7, except that the motions of the core and outer punches are respectively controlled in reverse.

Next, a description will be given in detail of an embodiment of the apparatus corresponding to the manufacturing method (Figs. 2) together with operations of the portions thereof mainly with reference to Figs. 5 and 6 and, as necessary, Figs. 2 as the apparatus for manufacturing the controlled release molded product of the present invention that is the rotary compression molding machine.

When viewed from above the turntable, the powder or granular particles supply portions 8, 9 and 10, powder or granular particles charging portions 11, 12 and 13, powder or granular particles rubbing-cutting portions 14, 15 and 16, compression molding portions 17, 18, 19 and 20, residual powder or granular particles removal portions 21 and 22 and a product unloading portion 23 are provided along the direction of rotation of a turntable 1 as shown in Fig. 5.

Description will be made on a mechanism-by-mechanism basis. The powder or granular particles supply portions (8, 9, 10 in Fig. 5) can be separated, according to the sequence of supply of powder or granular particles, into the portion 8 for supplying the powder or granular particles for the first outer layer OP1, the portion 9 for supplying the powder or granular particles for the cores NP and the portion 10 for supplying the powder or granular particles for the second outer layer OP2, with the powder or granular particles supplied by natural fall or by a metered supply machine (not shown) from hoppers 24, 25 and 26 filled with the respective powder or granular particles.

The respective powder or granular particles supplied by the powder or granular particles supply portions are sent next to the powder or granular particles charging portions (11, 12, 13 in Fig. 5). The powder or granular particles charging portions are designed to charge each of the powder or granular particles for the first outer layer OP1, the cores NP and the second outer layer OP2, respectively into the first outer layer space 201, the core space 202 or the second outer layer space 203 (refer to Figs. 2). These portions are intended to hold fixed amounts of the respective powder or granular particles supplied from the powder or granular particles supply portions using open feed shoes 27, 28 and 29, provided on the turntable 1 and capable of both storing and supplying the powder or granular particles, and introduce each of the powder or granular particles held by the feed shoes 27, 28 and 29 into the first outer layer space 201, the core space 202 or the second outer layer space 203 (refer to Figs. 2) by lowering the lower core punch 5A using lowerers 30, 31 and 32 provided on a frame 34, and in certain circumstances, by lowering the lower outer punch 5B using a lowerer 33 provided on a lower outer punch rail 36.

In detail, the powder or granular particles for the first outer layer OP1 is charged by lowering the lower core and outer punches 5A and 5B within the first open feed shoe 27 on the turntable 1 (Fig. 2A). Here, with the lower core and outer punches 5A and 5B lowered, the lower outer punch 5B is maintained at a constant height with respect to the turntable by moving the lower outer punch 5B, during charging of the powder or granular particles for the first outer layer OP1 or thereafter, along the lower outer punch rail 36 installed so as to bring the extreme tip portion of the lower outer punch 5B to the same height as the surface of the turntable 1 using the vertical sliding motion adjustment roller 73 of the lower outer punch (Fig. 2B). On the other hand, the lower core punch 5A is moved on a lower core punch rail 35 provided on the frame 34 using the lower core punch bottom portion 37 (substantially the same portion as the core punch compression head 79 in Figs. 7) and further adjusted to a predetermined position using the first core punch lowerer 30 provided on the lower core punch rail 35. The powder or granular particles for the first outer layer OP1 is thus introduced into the first outer layer space 201 roughly enclosed by the lower outer punch 5B and above the lower core punch 5A.

Next, the powder or granular particles for the cores NP is charged by raising the lower core punch 5A to a predetermined height within the second open feed shoe 28 on the turntable 1 and further moving the lower outer punch 5B on the lower outer punch rail 36 installed so as to bring the extreme tip portion of the lower outer punch 5B to the same height as the surface of the turntable 1 using the vertical sliding motion adjustment roller 73 of the lower outer punch (Figs. 2E to 2F). On the other hand, the lower core punch 5A holding the temporary molded product of the first outer layer on a lower core punch upper end surface 7A is moved using the lower core punch bottom portion 37 on the lower core punch rail 35 provided on the frame 34 and further lowered using the second core punch lowerer 31 provided on the lower core punch rail 35. The powder or granular particles for the cores NP is thus introduced into the core space 202 roughly enclosed by the lower outer punch 5B and above the temporary molded product of the first outer layer.

Further, the powder or granular particles for the second outer layer OP2 is charged by lowering both the lower core punch 5A holding the temporarily molded first outer layer OP1 and cores NP and the lower outer punch 5B or the lower outer punch 5B within the third open feed shoe 29 on the turntable 1 (Figs. 2I and 2J). Here, the lower outer punch 5B is lowered using the lower outer punch lowerer 33 provided on the lower outer punch rail 36. On the other hand, the lower core punch 5A is moved using the lower core punch bottom portion 37 on the lower core punch rail 35 provided on the frame 34 and lowered using the third core punch lowerer 32 provided on the lower core punch rail 35. The powder or granular particles for the second outer layer OP2 is thus introduced into the second outer layer space 203 created above and around the temporary molded products of the first outer layer OP1 and the cores NP within the die 3 by lowering both the lower core and outer punches 5A and 5B or only the lower outer punch 5B.

Although the third open feed shoe 29 is shown larger than the other open feed shoes in Fig. 6, this is intended to provide a detailed description thereof. It should be noted that, in place of the open feed shoes, agitation feed shoes may be employed that forcefully charge the powder or granular particles into the die using agitation vanes (installed at the same positions as the open feed shoes; not shown).

The die and punches charged with the powder or granular particles by the powder or granular particles charging portions next enter the powder or granular particles rubbing-cutting portions (14, 15, 16 in Fig. 5). The powder or granular particles rubbing-cutting portions adjust the powder or granular particles for the first outer layer OP1, the cores NP and the second outer layer OP2 supplied and charged as described above to fixed amounts. That is, the respective excess powder or granular particles overflowing from the given spaces are rubbed and cut for removal by rubbing-cutting plates 38, 39 and 40 as the lower core punch 5A or both the lower core and outer punches 5A and 5B are raised to predetermined positions by the lower outer and core punch rails 36 and 35.

In detail, the powder or granular particles for the first outer layer OP1 is rubbed and cut by the rubbing-cutting plate 38 attached to the first open feed shoe 27 on the turntable 1. Here, with the extreme tip portion of the lower outer punch 5B level with the surface of the turntable 1, the lower core punch 5A is raised to a predetermined position, thus causing the excess amount of the powder or granular particles for the first outer layer OP1 charged into the first outer layer space 201 to overflow from the space. Further, the overflowing powder or granular particles for the first outer layer OP1 is rubbed and cut by the rubbing-cutting plate 38 attached to the open feed shoe 27, thus leaving behind a fixed amount of the charged powder or granular particles for the first outer layer OP1 (prior to and following Fig. 2B).

Next, the powder or granular particles for the cores NP are rubbed and cut by the rubbing-cutting plate 39 attached to the second open feed shoe 28 on the turntable 1 as with the powder or granular particles for the first outer layer. Here, with the extreme tip portion of the lower outer punch 5B level with the surface of the turntable 1, the lower core punch 5A is raised to a predetermined position, thus causing the excess amount of the powder or granular particles for the cores NP charged into the core space 202 to overflow from the space. Further, the overflowing powder or granular particles for the cores NP is rubbed and cut by the rubbing-cutting plate 39 attached to the second open feed shoe 28, thus leaving behind a fixed amount of the charged powder or granular particles for the cores NP (prior to and following Fig. 2F).

The powder or granular particles for the second outer layer OP2 are also rubbed and cut by the rubbing-cutting plate 40 attached to the third open feed shoe 29 on the turntable 1 as with the powder or granular particles for the first outer layer and the cores. Here, the temporary molded products of the first outer layer and the cores, held by the lower core and outer punches 5A and 5B, are pushed up into the powder or granular particles for the second outer layer OP2 supplied into the die 3 as the lower core punch 5A or both the lower core and outer punches 5A and 5B are raised to predetermined positions, thus causing the excess amount of the powder or granular particles for the second outer layer OP2 to overflow. Further, the overflowing powder or granular particles for the second outer layer OP2 is rubbed and cut by the rubbing-cutting plate 40 attached to the third open feed shoe 29, thus leaving behind a fixed amount of the charged powder or granular particles for the second outer layer OP2 (following Fig. 2K).

The die and punches charged with fixed amounts of the powder or granular particles next enter the compression molding portions (17, 18, 19, 20 in Fig. 5). The compression molding portions are intended to perform precompression or main compression on one of the powder or granular particles for the first outer layer OP1, the cores NP and the second outer layer OP2 or a combination of two or more thereof (including temporary molded products) using compression rollers (44 to 51, 67 to 70) held by the frame 34.

In detail, temporary compression of the powder or granular particles for the first outer layer OP1 or the temporary molded product of the first outer layer OP1 and the powder or granular particles for the cores NP is carried out by pressing using the upper and lower core punches 4A and 5A. Here, the upper core punch 4A is lowered by upper core punch lowering cams 41 and 42 furnished on an upper core punch rail 52, and preferably the upper outer punch 4B is also concurrently lowered to a predetermined position by upper outer punch lowering cams 53 and 54 furnished on an upper outer punch rail 56, thus inserting the tip of the upper core punch 4A into the spaces above the lower core punch 5A and roughly enclosed by the lower outer punch 5B within the die 3. The powder or granular particles for the first outer layer OP1 charged into the given space or the temporary molded product of the first outer layer OP1 and the powder or granular particles for the cores NP are thus confined from above and below and pressed so as to be sandwiched between the upper temporary compression rollers 44 and 46 and the lower temporary compression rollers 45 and 47, thus molding temporary molded products (Figs. 2C and 2G). It should be noted that although not preferred, it is possible to omit the compression-molding portion for the powder or granular particles for the first outer layer OP1 provided at the beginning.

Precompression (temporary compression) of the temporary molded products of the first outer layer OP1 and the core NP and the powder or granular particles for the second outer layer OP2 is carried out by pressing using the upper core and outer punches 4A and 4B (upper punch) and the lower core and outer punches 5A and 5B (lower punch). To insert the upper core and outer punches 4A and 4B into the die 3, the upper core and outer punches 4A and 4B are lowered to predetermined positions using an upper core punch lowering cam 43 furnished on the upper core punch rail 52 and an upper outer punch lowering cam 55 furnished on the upper outer punch rail 56, inserting the tips thereof into the die 3. The temporary molded products of the first outer layer OP1 and the core NP and the powder or granular particles for the second outer layer OP2 are thus confined so as to be sandwiched from above and below and press-molded in a preliminary fashion by the precompression roller 48 for the upper core punch, the precompression roller 67 for the upper outer punch, a precompression roller 49 for the lower core punch and the precompression roller 68 for the lower outer punch.

In main compression following precompression (temporary compression), the aforementioned molded product press-molded in a preliminary fashion is press-molded as is in a full scale manner by the main compression roller 50 for the upper core punch, the main compression roller 69 for the upper outer punch, the main compression roller 51 for the lower core punch and the main compression roller 70 for the lower outer punch (Fig. 2M). It should be noted that although not preferred, it is possible to use only the present main compression portion by omitting the precompression portion of the molded products of the first outer layer OP1 and the core NP and the powder or granular particles for the second outer layer OP2.

Next, the residual powder or granular particles removal portions (21, 22 in Fig. 5) are provided at the precompression portion of the powder or granular particles for the first outer layer OP1 or the cores NP or a portion immediately thereafter. As shown in Figs. 2, in the temporary compression step or immediately thereafter, the lower outer punch 5B is held such that the extreme tip portion thereof is maintained at the same height as the surface of the turntable 1, and the powder or granular particles 57 for the first outer layer OP1 or the powder or granular particles 58 for the cores, remaining on the upper end surface 7B of the lower outer punch is removed by suction and/or compressed air injection, etc.

In detail, the upper end surface 7B of the lower outer punch 5B shown in Figs. 2 corresponds to the circumferential edge 76 (bevel angle) of the finished product shown in Figs. 3, and the residual powder or granular particles 57 and 58 remain at the portion. The residual powder or granular particles 57 and 58 are impossible to remove by rubbing and cutting using the rubbing-cutting plates 38 and 39 of the open feed shoes or agitation feed shoes provided on the turntable 1 and, if left unremoved, cause a concern over contamination between the powder or granular particles for the first outer layer OP1 and the cores NP and that between the powder or granular particles for the cores NP and the second outer layer OP2. In the embodiment, for this reason, the residual powder or granular particles 57 and 58 are removed by the first and second residual powder or granular particles removal portions 21 and 22 furnished on the turntable 1 following the precompression step (Fig. 2D and 2H). A residual powder or granular particles removal mechanism constituting the residual powder or granular particle removal portion comprises, for example as shown in Figs. 8, compressed air injection nozzles 60 for injecting compressed air onto the die surface from all directions and suction boxes 75 and 61 provided with suction holes 59 for aspirating the residual powder or granular particles, with the compressed air injection nozzles 60 and the suction boxes 75 and 61 arranged on and parallel to the surface of the turntable 1 so as to sandwich the die and the punches. The compressed air injection nozzles 60 inject compressed air onto the punches and the die from all directions and further the suction holes 59 near the die surface aspirate the residual powder or granular particles 57 and 58, keeping the residual powder or granular particles from flying outside for reliable removal thereof.

An alternative method of removing the residual powder or granular particles is by raising the upper core punch 4A or the upper core and outer punches 4A and 4B with the temporary molded product held in the space inside the lower outer punch 5B and aspirating the entire die from the upper end surface of the die (from the direction perpendicular to the turntable), thus removing the powder or granular particles for the first outer layer OP1 or for the cores NP remaining on the upper end surface 7B of the lower outer punch and/or the temporary molded product. It is mandatory in this method that the temporary molded product not be drawn in by suction, and temporary compression operation cannot be omitted from the outer layer molding step and the outer layer/core molding step. It should be noted that the present residual powder or granular particle removal portions may be omitted under certain circumstances. In particular, when a flat-surfaced molded product is made, the outer punch surface is also flat, thus requiring no residual powder or granular particle removal portions.

The final molded product is sent to the product-ejecting portion (23 in Fig. 5) for ejection outside the molding apparatus. The product eject-ejecting portion is designed to eject the product using a scraper 71 that guides to a chute 72 by pushing up the product as the lower core and outer punches 5A and 5B rise.

In detail, the upper core and outer punches 4A and 4B are raised along the rising sloped surface by upper core and outer punch raising cams 62 and 63, thus pulling the punch tips out of the die 3. Further, using lower core and outer punch push-up rails 66 and 65, the lower core and outer punches 5A and 5B are pushed up, thus completely pushing a molded product 64 out of the die 3. Here, it is preferred for easy ejecting of the molded product that the tip surface of the lower outer punch 5B be maintained at the same level as the surface of the turntable 1 and that the lower core punch 5A be pushed up slightly more upward than the tip surface of the lower outer punch 5B (Fig. 2N). The molded product 64 that has been pushed out is scraped using the scraper 71 for ejecting outside the turntable 1 and then guided into the chute 72 for retrieval of the product.

In the apparatus of the present invention shown in Fig. 6, means for moving the core and outer punches refer to the rails (the lower outer punch rail 36, the lower core punch rail 35, the upper outer punch rail 56, the upper core punch rail 52), the lowerers (the first core punch lowerer 30, the second core punch lowerer 31, the third core punch lowerer 32, the lower outer punch lowerer 33), the raising cams (the upper core and outer punch raising cams 62 and 63), the lowering cams (the upper core punch lowering cams 41, 42 and 43, the upper outer punch lowering cams 53, 54 and 55), the push-up rails (the lower core and outer punch push-up rails 66 and 65), the vertical sliding motion adjustment rollers (the vertical sliding motion adjustment rollers 73 and 74 of the lower and upper outer punches), the core punch bottom portion 37 and the bearings 77. On the other hand, means for allowing manipulation of the core and outer punches for compression operation refer to the compression rollers (the upper temporary compression rollers 44 and 46, the lower temporary compression rollers 45 and 47, the preliminary compression roller 48 for the upper core punch, the preliminary compression roller 67 for the upper outer punch, the precompression roller 49 for the lower core punch, the preliminary compression roller 68 for the lower outer punch, the main compression roller 50 for the upper core punch, the main compression roller 69 for the upper outer punch, the main compression roller 51 for the lower core punch, the main compression roller 70 for the lower outer punch), and the outer punch compression head 78 and the core punch compression head 79 as shown in Figs. 7. It should be noted that these include not only elements of the apparatus main body but also those of the punches.

### [Embodiments]

The controlled release molded product according to the present invention will be described specifically below with reference to the following embodiments.

### Test Example 1

### [Manufacturing Example 1]

The punch used, a flat edge angle punch capable of pressing, has a double structure consisting of a core punch whose three tip portions, rectangular or square, are arranged roughly at 1mm-intervals from the outside to the center portion and measure 2mm x 3mm, 1mm x 3mm and 1mm x 1mm on each side, and an outer punch of 10.0mm in outer diameter enclosing the outer perimeter of the core punch. A die provided with the aforementioned punch above and below the die was used for the following operations. Each punch surface was coated with a slight amount of magnesium stearate (TAIHEI CHEMICAL INDUSTRIAL). With the lower core punch lowered, 32mg, 16mg and 8mg of hydroxypropylcellulose (NIPPON SODA: HPC-L) were supplied into respective spaces enclosed by the lower outer punch and above the lower core punch from outside the molded product toward the center portion. By moving the upper and lower core punches to engage each other, precompression was manually conducted to such an extent that the surface became flat. Next, with the lower core punch lowered, 8mg, 4mg and 2mg of thoeophyline (SHIZUOKA COFFEIN: "The Japanese Pharmacopoeia" thoeophyline) were supplied into spaces above the temporary molded products of hydroxypropylcellulose and enclosed by the lower outer punch from outside toward the center portion. By moving the upper and lower core punches to engage each other, temporary compression was manually conducted similarly as previously done to such an extent that the surface became flat. Further, with the lower punch lowered, the remaining 352mg of hydroxypropylcellulose was supplied into a space above and around the molded products made up of hydroxypropylcellulose and thoeophyline within the die such that the temporary molded product of thoeophyline was completely covered by hydroxypropylcellulose. Then, by moving the upper and lower punches to engage each other, tabletting was conducted at a compression pressure of about 0.5 tons - this time using a hydraulic manual press (Shimadzu: SSP-10A). The tablet weight and hardness were found to be 421mg per tablet and 13kg, respectively.

### [Release Property Evaluation]

The molded product from manufacturing example 1 was evaluated for release property in conformance with the Dissolution Test of the General Tests, Processes and Apparatus of the Japanese Pharmacopoeia, Thirteenth Edition. It should be noted that pure water was used as test solution and that, to prevent the tablet from adhering to the test vessel, the molded product was placed in a Japanese Pharmacopoeia-compliant sinker for dissolution test. The amount of thoeophyline eluted was calculated by measuring, with a flow cell UV system (Shimadzu: UV-1600), the absorbance of given amounts of the sampling solution using a dissolution tester (TOYAMA CHEMICAL: NTR-6100A). The results are shown in Fig. 10.

It was discovered from Fig. 10 that there were three release peaks at three and half, six and half and nine hours. Further, it was found that, as compared with the first peak area, the second and third peak areas declined. The reason for this lies in that amount of thoeophyline released observed was proportional to the amount of thoeophyline contained in the cores. It was determined that the difference in core size (amount of thoeophyline added) was reflected in the release property. Although thoeophyline dissolution was observed in the interim period between cores, that is, while filler dissolution was in progress over a time period in the neighborhood of seven to eight hours after the start of dissolution, this was thought to be caused by effective ingredient diffusion through gel matrix because hydroxypropylcellulose, which was used to form the gel matrix was used as filler in the present test.

Thus, the inventor arrived at a molded product capable of readily controlling not only time-dependent release but also release amount by not providing multi-layer coating and by adjusting core positions and shapes.

### Test Example 2

While, in test example 1, a dissolution test was conducted using pure water (dissolution test at pH near neutral), an evaluation was performed next in test conditions assuming gastric juice and intestinal conditions using a so-called enteric filler (for example, Eudragit) that dissolves in the intestines rather than in the stomach.

### [Manufacturing Example 2]

The punch used, a flat edge angle punch capable of pressing, has a double structure consisting of a core punch whose three tip portions of 1.5mm in diameter are uniformly arranged (B-2 type in Figs. 1) from the outside toward the center portion of the punch and an outer punch of 10.0mm in outer diameter enclosing the outer perimeter of the core punch. A die provided with the aforementioned punch above and below the die was used for the following operations. Each punch surface was coated with a slight amount of magnesium stearate (same as above). With the lower core punch lowered, 20mg each of Eudragit (Eudragit L100-55: HIGUCHI) was supplied into respective spaces enclosed by the lower outer punch and above the lower core punch, and by moving the upper and lower core punches to engage each other, precompression was manually conducted similarly to such an extent that the surface became flat. Next, with the lower core punch lowered, 10mg, 6mg and 8mg of thoeophyline (same as above) were supplied into spaces above the temporary molded products of Eudragit and enclosed by the lower outer punch from the outer layer side toward the center portion. By moving the upper and lower core punches to engage each other, precompression was manually conducted similarly as previously done to such an extent that the surface became flat. Further, with the lower punch lowered, the remaining 320mg of Eudragit was supplied into a space above and around the molded products made up of Eudragit and thoeophyline within the die such that the temporary molded product of thoeophyline was completely covered by Eudragit. Then, by moving the upper and lower punches to engage each other, tabletting was conducted at a compression pressure of about 0.4 tons - this time using a hydraulic manual press (same as above). The tablet weight and hardness were found to be 402mg per tablet and 14kg, respectively.

### [Release Property Evaluation]

The molded product from manufacturing example 2 was evaluated for release property in conformance with test example 1.

First, to evaluate the present preparation for dissolution property in the acid region (assuming gastric juice condition), the evaluation was conducted using a first solution (pH1.2) as the dissolution test solution (not shown). As a result, absolutely no dissolution was observed in the two-hour dissolution test using the first solution, making evident that the present preparation had sufficient resistance to gastric juice.

Next, to evaluate the dissolution property in the basic region (assuming intestinal condition), the evaluation was conducted using a second solution (pH6.8) as the dissolution test solution (Fig. 11). As a result, it was discovered that there were three release peaks at one and half, five and half and eight and half hours. It was further confirmed from the difference in magnitude between individual release peaks, release was proportional to the amount of thoeophyline present in the cores.

From the above, a molded product was successfully obtained that is capable of releasing the effective ingredient at constant intervals while having the function as enteric coating even in the present preparation by direct tabletting as in the conventional arts.

While detailed descriptions have been given of the controlled release molded product of the present invention, the technical scope of the present invention is not limited to the aforementioned embodiments.

### INDUSTRIAL APPLICABILITY

Unlike controlled release molded products made by conventional intricate manufacturing methods such as multi-layer coating, the present invention is a molded product in which substantially a plurality of cores containing an effective ingredient are localized, making it possible to mold the molded product in a single step from powder or granular particles. This not only ensures high production efficiency but also allows for the cores to be arranged at specific positions, reducing to the minimal possible level variations between finished molded products and thereby allowing for manufacture of quality-assured highly accurate molded products. Therefore, the present invention makes controlled release molded products that are industrially viable in various fields and is best suited notably to the field of pharmaceutical drugs where accuracy is demanded of molded products.

## Claims

1. A controlled release molded product comprising an outer layer and a plurality of cores, wherein the release of an effective ingredient, contained in the core portion, is controlled by varying the distance from the outside of the molded products to the individual core arranged at the specific position.

2. A controlled release molded product comprising an outer layer and a core, provided with a portion partially having a constriction, that can be substantially identified as a plurality of cores, wherein the release of an effective ingredient, contained in the core portion, is controlled by varying the distance from the outside of the molded product to the individual portion that can be identified as the core arranged at the specific position.

3. A dividable controlled release molded product comprising the plurality of controlled release molded products according to claim 1 or 2 coupled along a coupling portion, wherein the plurality of controlled release molded products can be separated along the coupling portion, and wherein the distance from the division surface to any core in any individual controlled release molded product of the plurality of controlled release molded products is sufficiently longer than the distance from an external reference plane on the molded product surface that stipulates the distance from the outside of the molded product to the core.

4. The controlled release molded product according to any one of claims 1 to 3, wherein the release of the effective ingredient can be further controlled by the property of a filler ingredient as a component of the outer layer.

5. The controlled release molded product according to claim 4, wherein the filler ingredient contained in the outer layer is a matrix-type sustained release base.

6. The controlled release molded product according to any one of claims 1 to 5, wherein no filler ingredient having a release delaying property is contained in the core.

7. The controlled release molded product according to any one of claims 1 to 6, having a plurality of pulsed release properties.

8. The controlled release molded product according to any one of claims 1 to 7, wherein the molded product is manufactured by a compression molding method alone.
